(19) Europäisches Patentamt
European Patent Office
Office européen des brevets

(11) Veröffentlichungsnummer: **0 274 988**
**A2**

(12) # EUROPÄISCHE PATENTANMELDUNG

(21) Anmeldenummer: **87810752.3**

(22) Anmeldetag: **14.12.87**

(51) Int. Cl.⁴: **C07D 417/14**, C07D 417/04, C07D 213/78, A01N 43/82

(30) Priorität: **18.12.86 CH 5059/86**

(43) Veröffentlichungstag der Anmeldung: **20.07.88 Patentblatt 88/29**

(84) Benannte Vertragsstaaten: **AT BE CH DE FR GB IT LI NL**

(71) Anmelder: **CIBA-GEIGY AG**
**Klybeckstrasse 141**
**CH-4002 Basel(CH)**

(72) Erfinder: **Farooq, Saleem, Dr.**
**Kirchackerstrasse 27**
**CH-4422 Arisdorf(CH)**
Erfinder: **Ehrenfreund, Josef, Dr.**
**Amselstrasse 11**
**CH-4123 Allschwil(CH)**
Erfinder: **Waespe, Hans-Rudolf, Dr.**
**Feldstrasse 86**
**CH-4123 Allschwil(CH)**

(54) **Neue Dihydrothiadiazole.**

(57) Es werden neue, 2-Pyridyl-4,5-dihydro-1,3,4-thiadiazole der Formel I

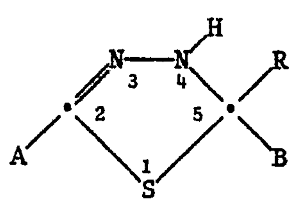

(I),

worin
A den 2-, 3-oder 4-Pyridylrest,
B Wasserstoff, $C_1$-$C_4$-Alkyl oder den 2-, 3-oder 4-Pyridylrest,
R Wasserstoff, $C_1$-$C_4$-Alkyl, Benzyl, unsubstituiertes oder durch Halogen, Nitro, $C_1$-$C_4$-Alkyl, $C_1$-$C_4$-Alkoxy, $C_1$-$C_4$-Alkoxy-$C_1$-$C_4$-alkyl oder Halogen-$C_1$-$C_4$-alkyl substituiertes Aryl bedeuten, beschrieben, sowie Verfahren zu ihrer Herstellung und ihre Verwendung in der Schädlingsbekämpfung.

EP 0 274 988 A2

Xerox Copy Centre

# Neue Dihydrothiadiazole

Die vorliegende Erfindung betrifft neue, gegebenenfalls substituierte 2-Pyridyl-4,5-dihydro-1,3,4-thiadiazole, Verfahren zu ihrer Herstellung und ihre Verwendung in der Schädlingsbekämpfung, sowie Mittel, welche diese Wirkstoffe enthalten.

Die vorliegende Erfindung bezieht sich auf neue 2-Pyridyl-4,5-dihydro-1,3,4-thiadiazole der Formel I

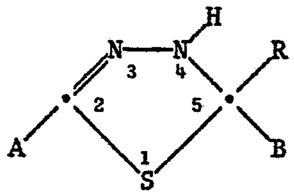

(I),

worin

A den 2-, 3-oder 4-Pyridylrest,

B Wasserstoff, $C_1$-$C_4$-Alkyl oder den 2-, 3-oder 4-Pyridylrest,

R Wasserstoff, $C_1$-$C_4$-Alkyl, Benzyl, unsubstituiertes oder durch Halogen, Nitro, $C_1$-$C_4$-Alkyl, $C_1$-$C_4$-Alkoxy, $C_1$-$C_4$-Alkoxy-$C_1$-$C_4$-alkyl oder Halogen-$C_1$-$C_4$-alkyl substituiertes Aryl bedeuten, sowie auf Salze und optische Isomeren einer Verbindung der Formel I.

Die für R sowohl für sich oder auch als Bestandteil anderer Reste stehenden $C_1$-$C_4$-Alkyle können verzweigt oder geradkettig sein. Beispiele solcher Alkyle sind u.a. Methyl und Aethyl sowie Propyl, Butyl und ihre Isomeren.

Bei dem unter R stehenden Aryl sind ein-oder mehrkernige aromatische Reste, insbesondere die unsubstituierten und substituierten Phenyl-und die 1-oder 2-Naphthylreste zu verstehen.

Bei den unter R genannten Halogenen handelt es sich sowohl um Fluor und Chlor als auch um Brom und Jod, wobei Fluor und Chlor bevorzugt sind. Diese Definition für Halogen gilt auch für die $C_1$-$C_4$-Halogenalkyle und Halogenaryle. Beispiele solcher Halogenalkyle sind u.a. das ein-bis dreifach durch Fluor, Chlor und/oder Brom substituierte Methyl, das ein-bis fünfach durch Fluor, Chlor und/oder Brom substituierte Aethyl oder das ein-bis siebenfach durch Fluor, Chlor und/oder Brom substituierte Propyl. Beispiele für Halogenaryle sind ein-bis dreifach durch Fluor, Chlor und/oder Brom substituierte Phenylreste.

Der vorliegende Erfindungsgegenstand umfasst weiterhin die Salze, insbesondere die pflanzenphysiologisch unbedenklichen Salze, der Verbindungen der Formel I. Als derartige Salze mit organischen und anorganischen Säuren kommen z.B. die folgenden in Betracht: Chloride, Bromide, Jodide, Sulfate, Hydrogensulfate, Chlorate, Perchlorate, Rhodanide, Nitrate, Phosphate, Hydrogenphosphate, Tetrafluorborate, Formiate, Acetate, Trichloracetate, Trifluoracetate, Phenylsulfonate, Oxalate, Malonate, Succinate, Malate, Tartrate oder Citrate. Von diesen Salzen sind die Bromide hervorzuheben.

Der vorliegende Erfindungsgegenstand umfasst weiterhin die optischen Isomeren der Verbindungen der Formeln I, die aus den racemischen Formen in an sich bekannter Weise mit Hilfe der üblichen Trennungsmethoden erhalten werden können.

Bevorzugt werden erfindungsgemäss Verbindungen der Formel I, worin B den 2-, 3-oder 4-Pyridylrest und R Wasserstoff oder $C_1$-$C_4$-Alkyl bedeuten.

Insbesondere bevorzugt sind von diesen Verbindungen jene, bei welchen A den 3-Pyridylrest und R Wasserstoff bedeuten.

Von dieser Gruppe ist das 2,5-Bis-(3-Pyridyl)-4,5-dihydro-1,3,4-thiadiazol, sowie dessen Salze als bedeutend hervorzuheben.

Von den erfindungsgemässen Verbindungen der Formel I sind auch jene zu erwähnen, bei welchen B den 2-, 3-oder 4-Pyridylrest, R Wasserstoff und A entweder den 2-oder den 4-Pyridylrest bedeuten.

Ebenfalls zu erwähnen sind jene Verbindungen der Formel I, bei welchen A den 3-oder 4-Pyridylrest, B Wasserstoff und R unsubstituiertes oder durch Halogen, Methyl, Aethyl oder Trifluormethyl monosubstituiertes Phenyl bedeutet.

Von dieser Gruppe weisen jene Verbindungen eine besondere Bedeutung auf, bei welchen A den 3-Pyridylrest und R den Rest

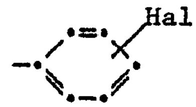

bedeuten.

Von diesen Halogenverbindungen ist das 2-(3-Pyridyl)-5-(4-chlorphenyl)-4,5-dihydro-1,3,4-thiadiazol als Einzelverbindung hervorzuheben.

Die Verbindungen der Formel I können in an sich bekannter Weise (vgl. D.M. Evans et al., J.Chem.Soc., Chem. Commun. 1982, 188; K.N. Zelenin et al., Khim. Geterotsikl. Soedin, 1982 No. 7, 904) hergestellt werden durch Umsetzung einer Verbindung der Formel II

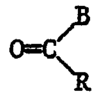

(II)

mit einer Verbindung der Formel III

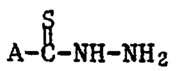

(III),

wobei in den Formeln II und III A, B und R die vorstehend unter Formel I angegebenen Bedeutungen haben (Verfahren a).

Die VErbindungen der Formel I können weiterhin dadurch hergestellt werden, dass man eine Verbindung der Formel IV

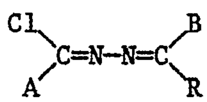

(IV),

worin A, B und R die vorstehend unter Formel I angegebenen Bedeutungen haben, mit einem Sulfid umsetzt (Verfahren b). Die Verbindungen der Formel IV sind in Analogie zu bekannten Methoden (vgl. J. Chem. Soc., Perkin Trans., 1 (2), 349-55; 1981) herstellbar.

Gegebenenfalls kann man eine wie vorstehend angegeben erhaltene Verbindung der Formel I in an sich bekannter Weise in eines ihrer Salze überführen.

Das zu den erfindungsgemässen Verbindungen der Formel I führende Verfahren a) wird vorzugsweise in einem Lösungsmittel vorgenommen. Geeignete Lösungsmittel sind z.B. aromatische Kohlenwasserstoffe, wie Benzol, Toluol und Xylol; Ketone, wie Aceton, Cyclohexanon und Methyläthylketon; Aether, wie Tetrahydrofuran, Dioxan und Diäthyläther; halogenierte Kohlenwasserstoffe, wie Chloroform, Tetrachlorkohlenstoff und Chlorbenzol; Alkohole, wie Aethanol und Propanol; Ester aliphatischer Säuren, wie Aethylacetat; aliphatische Amide, wie Dimethylformamid und Dimethylacetamid; Dimethylsulfoxid und andere Lösungsmittel, die die Reaktion nicht beeinträchtigen. Diese Lösungsmittel können auch als Gemische verwendet werden. Die Reaktionstemperatur kann in einem weiten Bereich von -10 bis +200°C liegen. Bevorzugt wird ein Temperaturbereich von Raumtemperatur bis etwa 150°C.

Verfahren b) wird vorzugsweise in einem polaren Lösungsmittel, wie Wasser, einem Alkohol, z.B. Methanol oder Aethanol, durchgeführt. Die Ringschlussreaktion wird mit einem Sulfid, vorzugsweise einem löslichen Metallsufid, wie Natrium-oder Kaliumsulfid, oder einem entsprechenden Hydrogensulfid, z.B. Kaliumhydrogensulfid, vorgenommen, wobei ein alkalisches Milieu bevorzugt wird. Die Reaktionstemperatur kann im Bereich von -15 bis +100°C liegen. Bevorzugt wird ein Temperaturbereich von -10 bis +50°C, z.B. Raumtemperatur.

Die als Ausgangsstoffe verwendeten Carbonylverbindungen der Formel II und Pyridinthiocarbonsäurehydrazide der Formel III sind bekannt oder können analog bekannten Verfahren erhalten werden. Die substituierten 2,3-Diazabutadiene der Formel IV sind analog bekannter Arbeitsweisen (vgl. J.Chem.Soc., Perkin. Trans., 1 (2), 349; 1981) wie folgt zugänglich, wobei $R_1$ und $R_2$ die vorstehend angegebenen Bedeutungen haben:

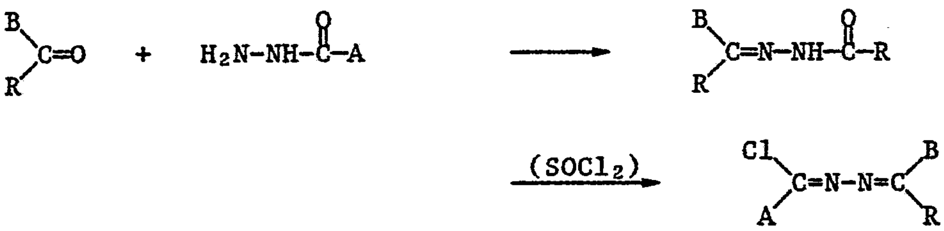


(IV) .

Von den substituierten Butadienen der Formel IV sind jene der Formel IVa

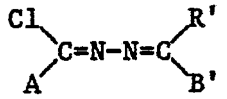

(IVa),

worin
A und B′ voneinander unabhängig den 2-, 3-oder 4-Pyridylrest und R′ Wasserstoff oder $C_1$-$C_4$-Alkyl bedeuten, neu. Die Verbindungen der Formel IVa bilden ebenfalls einen Gegenstand der vorliegenden Erfindung.

Es wurde gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Schädlingen.

Insbesondere eignen sich die Verbindungen der Formel I zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen der Formel I entspricht einer Abtötungsrate (Mortalität) von mindestens 59-60 % der erwähnten Schädlinge.

Mit Hilfe der erfindungsgemässen Verbindungen der Formel I können vor allem pflanzenschädigende Insekten, speziell pflanzenschädigende Insekten in Zier-und Nutzpflanzungen, insbesondere in Baumwollkulturen, Gemüsekulturen, Reiskulturen und Obstkulturen bekämpft werden. In diesem Zusammenhang ist hervorzuheben, dass die genannten Verbindungen sich sowohl durch eine stark ausgeprägte systemische als auch Kontakt-Wirkung gegen saugende Insekten, vor allem gegen Insekten der Familie Aphididae (wie z.B. Aphis fabae, Aphis craccivora und Myzus persicae), welche sich mit herkömmlichen Mitteln nur - schwierig bekämpfen lassen, auszeichnen.

Die Verbindungen der Formel I zeichnen sich weiterhin durch eine gute Wirkung gegen larvale Insektenstadien und Nymphen, speziell von fressenden Schadinsekten, aus. Insbesondere können die Verbindungen der Formel I mit ausgezeichnetem Erfolg gegen pflanzenschädigende Zikaden, speziell in Reis-Kulturen, eingesetzt werden.

Die Verbindungen der Formel I eignen sich ferner zur Bekämpfung von Ektoparasiten, z.B. Lucilia sericata, sowie von Zecken an Haus-und Nutztieren, z.B. durch Tier-, Stall-und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreiten und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächnaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl-oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl-oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali-oder gegebenenfalls substituierten Ammoniumsalze von höheren Fettsäuren ($C_{10}$-$C_{22}$), wie z.B. die Na-oder K-Salze der Oel-oder Stearinsäure, oder von natürlichen Fettsäuregemischen, die z.B. aus Kokosnuss-oder Tallöl gewommen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali-oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca-oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 28 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolyporpylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zübereitungen enthalten - auf das Gewicht bezogen -in der Regel 0,1 bis 99 %, insbesondere 0,1 bis 95 %, eines Wirkstoffes der Formel I oder Kombinationen davon mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Beispiel H1: Herstellung von 3-Pyridincarbonyl-pyridin-3-aldehydhydrazon.

Zu einer Lösung von 41,1 g (0,3 M) Nikotinsäurehydrazid und 32,1 g (0,3M) Pyridin-3-aldehyd in 300 ml Aethanol werden bei Raumtemperatur fünf Tropfen Eisessig zugegeben. Das aus dem Reaktionsgemisch nach 2 Stunden ausgefallene Produkt wird abfiltriert und mit 100 ml Aethanol gewaschen. Nach Trocknen erhält man das 3-Pyridincarbonyl-pyridin-3-aldehyd-hydrazon mit einem Schmelzpunkt von 213°-216°C.

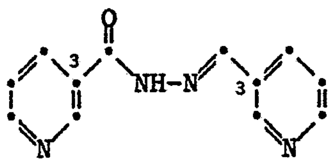

Beispiel H2: Herstellung von 1-Chlor-1,4-bis-(pyrid-3-yl)-2,3-diazabutadien (Verbindung 4.1)

Eine Suspension von 60,2 g (0,266 M) 3-Pyridincarbonyl-pyridin-3-aldehyd-hydrazon wird in 1100 ml kochendem Toluol mit 94,9 g (0,798 M) Thionylchlorid umgesetzt. Das Lösungsmittel wird nach ca. achtstündigem Kochen im Vakuum verdampft, der Rückstand in 700 ml Tetrahydrofuran gelöst und mit 41 g Triäthylamin verrührt. Das Lösungsmittel wird nach 30 minütigem Rühren bei Raumtemperatur im Vakuum verdampft, und der Rückstand in Essigester gelöst. Die Lösung wird nach Waschen mit Wasser, und mit gesättigter Kochsalzlösung über $Na_2SO_4$ getrocknet und das Lösungsmittel wird verdampft. Der Rückstand wird mit heissem Tetrahydrofuran verrieben, abfiltriert, und getrocknet. Man erhält das 1-Chlor-1,4-bis-(pyrid-3-yl)-2,3-diazabutadien mit einem Schmelzpunkt von 103°-105°C.

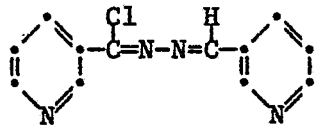

Beispiel H3: Herstellung von 2,5-Bis-(pyrid-3-yl)-4,5-dihydro-1,3,4-thiadiazol. (Verbindung Nr. 1.1)

Eine mit Schwefelwasserstoff frisch gesättigte Lösung von 6,7 g (0,1 M) 85%iger Kaliumhydroxyd in 190 ml Aethanol wird mit 24,3 g (0,1 M) 1-Chlor-1,4-bis-(pyrid-3-yl)-2,3-diazabutadien in der Kälte versetzt. Nach 2 Stunden wird das Lösungsmittel im Vakuum verdampft, der Rückstand in Essigester gelöst, die

Essigester-Lösung mit Wasser, mit gesättigter Kochsalzlösung gewaschen, dann über Natriumsulfat getrocknet, filtriert und eingedampft. Der Rückstand wird mit Hexan/Aether verührt, filtriert, gewaschen und getrocknet. Man erhält auf diese Weise das 2,5-Bis-(pyrid-3-yl)-4,5-dihydro-1,3,4-thiadiazol mit einem Schmelzpunkt von 77°-78°C.

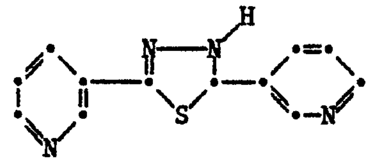

.

Beispiel H4:

a) Herstellung von 1-Nicotinoyl-2-(4-chlorbenzyliden)-hydrazin: Zu einer Lösung von 27,4 g Nikotinsäurehydrazid und 28,1 g 4-Chlorbenzaldehyd in 300 ml Aethanol werden bei Raumtemperatur fünf Tropfen Eisessig zugegeben. Die unter schwacher Wärmeentwicklung entstandene Suspension wird zwei Stunden bei Raumtemperatur gerührt, das ausgefallene Produkt abfiltriert und mit ca. 100 ml Aethanol gewaschen. Nach dem Trocknen erhält man die Titelverbindung mit einem Schmelzpunkt von 188°-190°C der Formel

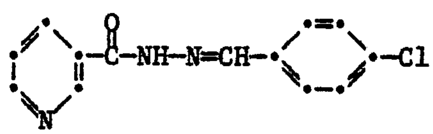

.

b) Herstellung von 1-Chlor-1-(pyrid-3-yl)-4-(4-chlorphenyl)-2,3-diazabutadien (Verbindung Nr. 4.2): Eine Suspension von 44 g des gemäss a) erhaltenen 1-Nicotinoyl-2-(4-chlorbenzyliden)-hydrazins in 700 ml Toluol wird am Rückfluss erhitzt. Zu dieser Suspension werden 60,5 g Thionylchlorid innerhalb etwa 1,5 Stunden zugetropft. Nach 6-stündigem Erhitzen am Rückfluss wird das klare, dunkelgelbe Reaktionsgemisch eingedampft. Der feste Rückstand wird in Essigester aufgeschlämmt und abfiltriert. Nach dem Trocknen erhält man die Titelverbindung als entsprechendes Hydrochlorid vom Schmelzpunkt 155°-160°C der Formel

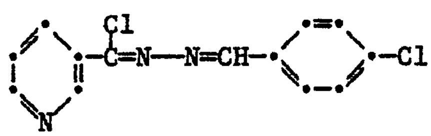

• HCl

.

c) Herstellung von 2-(Pyrid-3-yl)-5-(4-chlorphenyl)-4,5-dihydro1,3,4-thiadiazol (Verbindung Nr. 1.11): Eine Lösung von 13,8 g Kaliumhydroxyd in 150 ml Aethanol wird mit Schwefelwasserstoff gesättigt. Zu dieser Lösung werden 13,8 g Kaliumhydroxyd in 150 ml Aethanol zugetropft. Anschliessend wird die erhaltene Lösung portionsweise mit 38,7 g 1-Chlor-1-(pyrid-3-yl)-4-(4-chlorphenyl)-2,3-diazabutadien-hydrochlorid (hergestellt gemäss b) unter Eiskühlung versetzt. Das Reaktionsgemisch wird während 1 Stunde bei Raumtemperatur nachgerührt, dann eingeengt und der verbleibende Rückstand in Essigester gelöst. Die Essigester-Lösung wird zweimal mit Wasser und zweimal mit gesättigter Kochsalzlösung gewaschen, über Natriumsulfat getrocknet, filtriert und eingedampft. Man erhält auf diese Weise die Titelverbindung der Formel

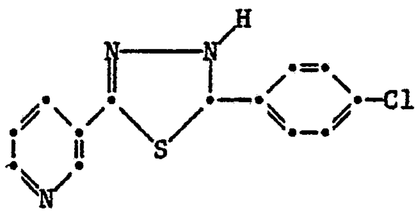

mit einem Schmelzpunkt von 108°-110°C (Verbindung Nr. 1.11).

Entsprechend den vorstehend beschriebenen Arbeitsweisen sind auch die folgenden Verbindungen der Formel I und der Formel IVa herstellbar:

Die Verbindungen Nr. 1.1 - 1.33 der Formel I und die Verbindungen Nr. 4.1 und 4.2. der Formel IVa der folgenden Tabellen 1 und 2 sind nach den beschriebenen Verfahren herstellbar.

Tabelle 1

Verbindungen der Formel I

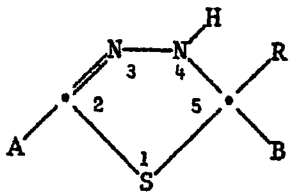

(I),

| Verb. Nr. | A | B | R | Physikalische Daten |
|---|---|---|---|---|
| 1.1 | Pyrid-3-yl | Pyrid-3-yl | H- | Schmp.77-78° |
| 1.2 | Pyrid-3-yl | Pyrid-2-yl | H- | |
| 1.3 | Pyrid-3-yl | Pyrid-4-yl | H- | |
| 1.4 | Pyrid-2-yl | Pyrid-2-yl | H- | |
| 1.5 | Pyrid-2-yl | Pyrid-3-yl | H- | |
| 1.6 | Pyrid-2-yl | Pyrid-4-yl | H- | |
| 1.7 | Pyrid-4-yl | Pyrid-2-yl | H- | |
| 1.8 | Pyrid-4-yl | Pyrid-3-yl | H- | |
| 1.9 | Pyrid-4-yl | Pyrid-4-yl | H- | |
| 1.10 | Pyrid-3-yl | Pyrid-3-yl | $CH_3$- | |
| 1.11 | Pyrid-3-yl | H | 4-Cl-$C_6H_4$ | Schmp.108-110° |
| 1.12 | Pyrid-3-yl | H | H | |
| 1.13 | Pyrid-3-yl | -$CH_3$ | H | |
| 1.14 | Pyrid-3-yl | -$CH_3$ | -$CH_3$ | |
| 1.15 | Pyrid-3-yl | Pyrid-3-yl | H | |
| 1.16 | Pyrid-3-yl | Pyrid-2-yl | H | |
| 1.17 | Pyrid-3-yl | Pyrid-4-yl | H | |
| 1.18 | Pyrid-3-yl | Pyrid-3-yl | $CH_3$ | |
| 1.19 | Pyrid-3-yl | H | 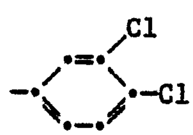 | Schmp.168-170° |
| 1.20 | Pyrid-3-yl | H | 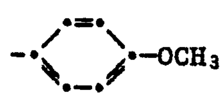 | Schmp.79-80° |
| 1.21 | Pyrid-3-yl | H | 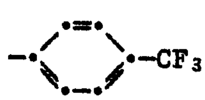 | |

| Verb. Nr. | A | B | R | Physikalische Daten |
|---|---|---|---|---|
| 1.22 | Pyrid-3-yl | H | 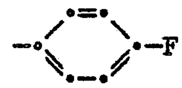 | Schmp.80-82° |
| 1.23 | Pyrid-3-yl | H | 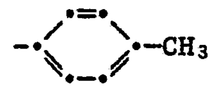 | Schmp.97-98° |
| 1.24 | Pyrid-3-yl | H | 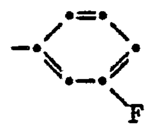 | Schmp.86-88° |
| 1.25 | Pyrid-3-yl | H | 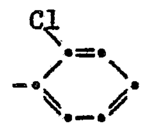 | Schmp.155-157° |
| 1.26 | Pyrid-3-yl | H | 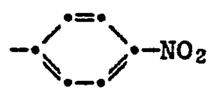 | |
| 1.27 | Pyrid-3-yl | H | 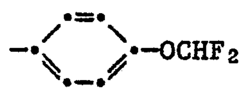 | |
| 1.28 | Pyrid-2-yl | H | 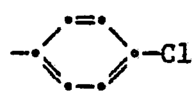 | |
| 1.29 | Pyrid-2-yl | H | 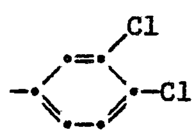 | |
| 1.30 | Pyrid-4-yl | H | 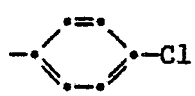 | |
| 1.31 | Pyrid-4-yl | H | 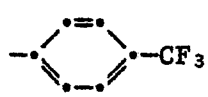 | |
| 1.32 | Pyrid-3-yl | Pyrid-3-yl | H | Oxalat Schmp.143-144° |
| 1.33 | Pyrid-4-yl | Pyrid-3-yl | H | Schmp.86-88° |

Tabelle 2

Verbindungen der Formel IVa

| Verb. Nr. | A | B' | R' | Physikalische Daten |
|---|---|---|---|---|
| 4.1 | Pyrid-3-yl | Pyrid-3-yl | H | Schmp.103-105° |
| 4.2 | Pyrid-3-yl | H | 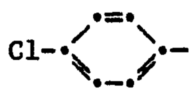 | Schmp.155-60° (Hydrochlorid) |
| 4.3 | | | | |
| 4.4 | | | | |

Formulierungsbeispiele

Formulierungen für Wirkstoffe der Formel I gemäss Tabelle 1 resp. Kombinationen dieser Wirkstoffe mit andern Insektiziden oder Akariziden (% = Gewichtsprozent):

| F1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

F2. Emulsions-Konzentrat

Wirkstoff oder Wirkstoffkombination 10 %
Octylphenolpolyäthylenglykoläther (4-5 Mol AeO) 3 %
Ca-Dodecylbenzolsulfonat 3 %
Ricinusölpolyglykoläther (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

| F3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

F4. Extruder-Granulat

Wirkstoff oder Wirkstoffkombination 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

F5. Umhüllungs-Granulat

Wirkstoff oder Wirkstoffkombination 3 %
Polyäthylenglykol (MG 200) 3 %
Kaolin 94 %

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

F6. Suspensions-Konzentrat

Wirkstoff oder Wirkstoffkombination 40 %
Aethylenglykol 10 %
Nonylpohenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Insektizide Kontaktwirkung: Myzus persicae

Etwa 4 cm hohe, in Wasser angezogene Erbsenkeimlinge werden vor Versuchsbeginn mit je ca. 200 Individuen von Myzus persicae besiedelt. Die so behandelten Pflanzen werden 24 Stunden später mit wässrigen Suspensionen enthaltend 200, 100, 50, 25, 12 und 6 ppm der Verbindung Nr. 1.11 bis zur Tropfnässe besprüht. Die Bonitur auf Mortalität erfolgt 48 Stunden nach Applikation. Der Versuch wird bei 20-22°C und 60 % relativer Luftfeuchtigkeit durchgeführt.

**Ergebnis:**

| | | | | | | |
|---|---|---|---|---|---|---|
| ppm | : 200 | 100 | 50 | 25 | 12 | 6 |
| % Mortalität: | 100 | 99 | 98 | 97 | 97 | 92 |

Beispiel B2: Wirkung gegen Musca domestica

Je 50 g frisch zubereitetes CSMA-Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 800 ppm ergibt. Nach dem Durchmischen des Substrates lässt man das Aceton mindestens 20 Stunden lang verdampfen. Dann werden pro Wirkstoff je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Tabelle I zeigen gute Wirkung im obigen Test.

Beispiel B3: Wirkung gegen Lucilia sericata (Larven)

Zu 9 ml eines Zucktmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Tabelle 1 zeigen in diesem Test gute Wirkung gegen Lucilia sericata.

Beispiel B4: Wirkung gegen Aedes aegypti (Larven)

Auf die Oberfläche von 150 ml Wasser, das sich in einem Behälter befindet, wird so viel einer 0,1%igen acetonischen Lösung des Wirkstoffes pipettiert, dass eine Konzentration von 400 ppm erhalten wird. Nach Verdunsten des Acetons wird der Behälter mit 30 bis 40 2-tägigen Aedes-Larven beschickt. Nach 2 und 7 Tagen wird die Mortalität geprüft.

Verbindungen der Formel I gemäss Tabelle 1 zeigen gute Wirkung im obigen Test.

Beispiel B5: Kontaktwirkung auf Aphis craccivora

In Töpfen angezogene 4-5 Tage alte Erbsenkeimlinge (Pisum sativum) werden vor Versuchsbeginn mit je ca. 200 Individuen von Aphis craccivora besiedelt. Die so behandelten Pflanzen werden 24 Stunden später mit einer wässrigen Zubereitung enthaltend 12,5 ppm der zu prüfenden Verbindung bis zur Tropfnässe besprüht. Man verwendet pro Test-Verbindung zwei Pflanzen. Bonitur auf Mortalität erfolgt nach 24 und 72 Stunden. Der Versuch wird bei 21-22°C und einer relativen Luftfeuchtigkeit von etwa 55 % durchgeführt.

Verbindung Nr. 1.11 gemäss Tabelle 1 zeigt in diesem Test eine Wirkung (Mortalität) von 80-100 %.

Beispiel B6: Systemische Wirkung auf Myzus persicae (Wasser)

Erbsenkeimlinge (2 cm), die 24 Stunden vor Beginn des Versuches mit einer Population von ca. 200 Individuen von Myzus persicae (R-Stamm) besiedelt worden sind, werden in 20 ml einer wässrigen Spritzbrühe, enthaltend 100 ppm des zu prüfenden Wirkstoffs, eingestellt. Die Spritzbrühe wird durch wässrige Verdünnung eines Emulsionskonzentrats oder eines Spritzpulvers hergestellt. Die Erbsenwurzel wird durch das zentrale Loch im Kunststoffdeckel des Testgläschens in die wässrige Wirkstoffbrühe eingeführt. Der Versuch wird bei 21°C und 60 % relativer Luftfeuchtigkeit durchgeführt. Nach 2, 3 und 6 Tagen wird auf % saugunfähige Blattläuse ausgewertet. Damit kann festgestellt werden, ob der über die Wurzel aufgenommene Wirkstoff die Insekten an den oberirdischen Pflanzenteilen abzutöten vermag.

Die Auswertung der erzielten Abtötung erfolgt 48 und 72 Stunden nach Versuchsbeginn. Pro Testsubstanz werden zwei Pflanzen, je eine in einem separatem Topf, verwendet. Der Versuch wird bei 25°C und ca. 70 % relativer Luftfeuchtigkeit durchgeführt.

Verbindungen der Formel I gemäss Tabelle 1 zeigen gute Wirkung in diesem Test.

Beispiel B7: Systemische Wirkung auf Myzus persicae

Peperonipflanzen im 4-bis 5-Blatt-Stadium in Töpfen mit 600 ccm Erde werden mit einer Population von 200-400 Myzus persicae besiedelt. Anschliessend werden 50 ml einer wässrigen Formulierung der zu prüfenden Verbindung (erhalten aus einem 25%igen Spritzpulver) oder Emulsionskonzentrat in einer Konzentration von 400 ppm direkt auf die Erde aufgegossen, ohne die Pflanze selbst zu benetzen. 3 und 6 Tage nach Behandlung wird auf Mortalität bonitiert.

Pro Testsubstanz werden zwei Pflanzen verwendet. Der Versuch wird bei ca. 22°C und 60 % relativer Luftfeuchtigkeit durchgeführt.

Verbindungen der Formel I gemäss Tabelle 1 zeigen gute Wirkung in obigem Test.

Beispiel B8: Blattpenetrations-Wirkung auf Aphis craccivora

In etwa 8 cm hohe Kunststoffbecher (Durchmesser etwa 6 cm) wird ein passender kleiner Zweig von Vicia faba gelegt, der mit Blattläusen der Spezies Aphis craccivora stark infiziert ist. Der Becher wird mit einem Kartondeckel, der in der Mitte eine ausgestanzte Oeffnung von 2.5 cm Durchmesser aufweist, bedeckt. Auf die in dem Deckel befindliche Oeffnung wird ein Blatt einer Vicia faba-Pflanze gelegt, ohne dieses Blatt von der eingetopften Pflanze abzutrennen. Das Blatt wird dann mit einem zweiten Kartonlochdeckel auf dem Becher über der Oeffnung des ersten Deckels fixiert. Von der Unterseite her, d.h. durch die Oeffnung des unteren Kartons hindurch, besiedeln nun die in dem Becher befindlichen Blattläuse das obenliegende Blatt der Futterpflanze. Auf der Oberseite wird auf das Blatt eine wässrige Zubereitung des zu prüfenden Wirkstoffes in einer Konzentration von 100 ppm mittels eines Pinsels gleichmässig aufgetragen. Es wird geprüft, ob die einseitig auf die Oberseite des Blattes der Futterpflanze aufgetragene Testsubstanz in genügender Menge in das Blattgewebe eindringt, damit es Blattläuse auf der Unterseite des Blattes abzutöten vermag.

Der Versuch wird bei etwa 22°C und 60 % relativer Luftfeuchtigkeit durchgeführt. Die Auswertung auf %-Mortalität erfolgt 48 Stunden nach Wirkstoffapplikation.

Verbindungen der Formel I gemäss Tabelle 1 zeigen gute Wirkung in obigem Test.

Beispiel B9: Frassfigt-Wirkung auf Laodelphax striatellus und Nilaparvata lugens (Nymphen)

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer acetonischen Lösung enthaltend 400 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem

Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Verbindungen der Formel I gemäss Tabelle 1 zeigen gute Wirkung in diesem Test.

Beispiel B10: Ovizide Wirkung auf Laodelphax striatellus und Nilaparvata lugens

Der Test wird an wachsenden Pflanzen durchgeführt. Jeweils 4 Reispflanzen (Dicke des Stengels 8 mm, Höhe ca. 20 cm) werden in Töpfe (Durchmesser von 8 cm) eingepflanzt.

Die Pflanzen werden auf einem Drehteller mit 100 ml einer acetonischen Lösung enthaltend 400 ppm des jeweiligen Wirkstoffes besprüht. Nach dem Antrocknen des Spritzbelages wird jede Pflanze mit 3 adulten Weibchen besiedelt. Um die Tiere am Entweichen zu hindern, wird über jede besiedelte Pflanze ein Glaszylinder gestülpt und dieser mit einem Gaze-Deckel abgedeckt. Die Weibchen bleiben 4 Tage zur Eiablage auf der behandelten Pflanze und werden dann entfernt.

Etwa 8 Tage nach Besiedelung schlüpfen die jungen Zikaden, und es erfolgt die Auszählung. Aus dem Vergleich der Anzahl geschlüpfter Larven auf den behandelten Pflanzen zu den auf unbehandelten Kontrollpflanzen geschlüpften Tieren wird die prozentuale Mortalität bestimmt.

Verbindungen gemäss Tabelle 1 zeigen in obigem Test gute ovizide Wirkung.

## Ansprüche

1. 2-Pyridyl-4,5-dihydro-1,3,4-thiadiazole der Formel I

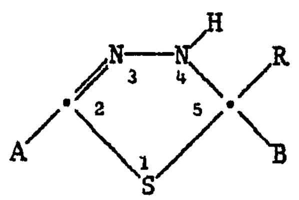

(I),

worin
A den 2-, 3-oder 4-Pyridylrest,
B Wasserstoff, $C_1$-$C_4$-Alkyl oder den 2-, 3-oder 4-Pyridylrest,
R Wasserstoff, $C_1$-$C_4$-Alkyl, Benzyl, unsubstituiertes oder durch Halogen, Nitro, $C_1$-$C_4$-Alkyl, $C_1$-$C_4$-Alkoxy, $C_1$-$C_4$-Alkoxy-$C_1$-$C_4$-alkyl oder Halogen-$C_1$-$C_4$-alkyl substituiertes Aryl bedeuten, sowie Salze und optische Isomeren einer Verbindung der Formel I.

2. Verbindungen gemäss Anspruch 1, dadurch gekennzeichnet, dass B den 2-, 3-oder 4-Pyridylrest und R Wasserstoff oder $C_1$-$C_4$-Alkyl bedeuten.

3. Verbindungen gemäss Anspruch 2 dadurch gekennzeichnet, dass A den 2-Pyridylrest und R Wasserstoff bedeuten.

4. Verbindungen gemäss Anspruch 2 dadurch gekennzeichnet, dass A den 3-Pyridylrest und R Wasserstoff bedeuten.

5. Das 2,5-Bis-(3-pyridyl)-4,5-dihydro-1,3,4-thiadiazol und dessen Salze gemäss Anspruch 4.

6. Verbindungen gemäss Anspruch 2 dadurch gekennzeichnet, dass A den 4-Pyridylrest und R Wasserstoff bedeuten.

7. Verbindungen gemäss Anspruch 1 dadurch gekennzeichnet, dass A den 3-oder 4-Pyridylrest, B Wasserstoff und R einen unsubstituierten oder durch Halogen, Methyl, Aethyl oder Trifluormethyl monosubstituierten Phenylrest bedeuten.

8. Verbindungen gemäss Anspruch 7 dadurch gekennzeichnet, dass A den 3-Pyridyrest und R einen halogensubstituierten Phenylrest bedeuten.

9. Das 2-(3-Pyridyl)-5-(4-chlorphenyl)-4,5-dihydro-1,3,4-thiadiazol nach Anspruch 8.

10. Verfahren zur Herstellung der Verbindungen der Formel I, Anspruch 1, dadurch gekennzeichnet, dass man
a) eine Verbindung der Formel II

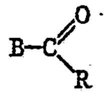

(II),

mit einer Verbindung der Formel III

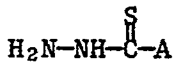

(III),

oder

b) eine Verbindung der Formel IV

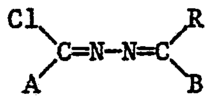

(IVa),

mit einem Sulfid umsetzt,

wobei A, B und R die im Anspruch 1 angegebene Bedeutung haben, und dass man gegebenenfalls eine erhaltene Verbindung der Formel I in eines ihrer Salze überführt oder in ihre optischen Isomeren trennt.

11. Verbindungen der Formel IVa,

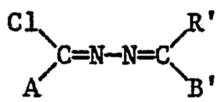

(IVa),

worin A und B′ voneinander unabhängig den 2-, 3-oder 4-Pyridylrest und R′ Wasserstoff oder $C_1$-$C_4$-Alkyl bedeuten.

12. Das 1-Chlor-1,4-bis-(pyridyl-3-yl)-diazabutadien nach Anspruch 11.

13. Schädlingsbekämpfungsmittel, welches als aktive Komponente eine Verbindung gemäss einem der Ansprüche 1 bis 9 zusammen mit geeigneten Trägern und/oder anderen Zuschlagstoffen enthält.

14. Verwendung einer Verbindung gemäss einem der Ansprüche 1 bis 9 zur Bekämpfung von Insekten und Vertretern der Ordnung Akarina an Tieren und Pflanzen.

15. Verwendung gemäss Anspruch 14 zur Bekämpfung von pflanzenschädigenden Insekten.

16. Verwendung gemäss Anspruch 15 zur Bekämpfung von pflanzenschädigenden saugenden Insekten.

17. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I bzw. Ia) gemäss einem der Ansprüche 1 bis 9 oder mit einem Mittel enthaltend neben Zusatz-und Trägerstoffen eine pestizid wirksame Menge dieser Verbindung, in Kontakt bringt oder behandelt.